# EUROPÄISCHE PATENTANMELDUNG

(11) **EP 1 743 668 A1**
(43) Veröffentlichungstag der Anmeldung: **17.01.2007**
(21) Anmeldenummer: 05405431.7
(22) Anmeldetag: 11.07.2005
(51) Int. Cl.: A61M 5/32

(54) **Sicherheitsvorrichtung für eine Injektionsnadel**

(71) Anmelder: Schwöbel, Eckhard, Dipl.-Ing., 6005 Luzern (CH)
(72) Erfinder: Schwöbel, Eckhard, Dipl.-Ing., 6005 Luzern (CH)
(74) Vertreter: EGLI-EUROPEAN PATENT ATTORNEYS

(57) **Zusammenfassung**

Eine Sicherheitsvorrichtung (2) für eine Injektionsnadel (1) mit einem an einem Halteteil (6) der Injektionsnadel (1) anbringbaren Basisteil (4) hat ein über ein elastisches Gelenkteil (5) mit dem Basisteil (4) verbundenes längliches Schutzteil (3). Vorzugsweise sind das Basisteil (4), das elastische Gelenkteil (5) und das Schutzteil (3) einstückig und aus Kunststoff hergestellt. Das Schutzteil (3) hat eine mechanisch wirkende Einrastvorrichtung in der Form eines überstehendes Sperrteils (9) für die Sicherung der Injektionsnadel (1) in einer Schutzposition. Das Schutzteil (3) ist mittels des elastischen Gelenkteiles (5) seitlich von der Längsachse der Injektionsnadel (1) wegklappbar. Das Schutzteil (3) ist zumindest in einem Teilbereich (7) seiner Längserstreckung längselastisch ausgebildet. Die Vorteile bestehen in einer einfach zu bedienenden und zuverlässig wirkenden Sicherheitsvorrichtung zum Schutz des medizinischen Personals.

## Beschreibung

Die Erfindung betrifft eine Sicherheitsvorrichtung für eine Injektionsnadel bzw. eine Injektionskanüle gemäss Patentanspruch 1.

Die Erfindung betrifft insbesondere eine Sicherheitsvorrichtung für eine Injektionsnadel, bei der ein Schutzteil, das zum Schutz vor Verunreinigungen der Nadel und zum Schutz des medizinischen Personals dient, zumindest die Spitze der Injektionsnadel umgibt und seitlich von der Längsachse der Injektionsnadel wegklappbar ist.

Sicherheitsvorrichtungen dieser Art werden gebraucht, um einerseits die Sterilität der Injektionsnadel vor dem Gebrauch sicherzustellen, andererseits aber auch um Verletzungen des Personals durch möglicherweise infizierte Injektionsspritzen nach dem Gebrauch zu verhindern.

Es gibt inzwischen eine Vielzahl derartiger Lösungen, es ist aber nach wie vor ein Bedarf nach einfach herstellbaren und einfach zu handhabenden Vorrichtungen dieser Art vorhanden.

So zeigt beispielsweise die US-4 664 259 eine gattungsgemässe Sicherheitsvorrichtung für eine Injektionsnadel. Diese Sicherheitsvorrichtung hat ein Basisteil, das mit einem Halteteil der Injektionsnadel verbunden ist. An diesem Basisteil ist über ein elastisches Gelenkteil ein längliches Schutzteil für die Injektionsnadel angebracht. Dabei sind das Basisteil, das elastische Gelenkteil (ein sog. ,living hinge') und das Schutzteil einstückig und aus Kunststoff hergestellt, und das Schutzteil hat zudem eine mechanisch wirkende Einrastvorrichtung in der Form eines einrastbaren Hakens zur Sicherung der Injektionsnadel in einer Schutzposition. Das Schutzteil ist somit mittels des elastischen Gelenkteiles seitlich von der Längsachse der Injektionsnadel wegklappbar und kann so von der Schutzposition in eine Gebrauchsposition, und umgekehrt, gebracht werden.

Weitere ähnliche Lösungen zeigen beispielsweise auch die US-4 982 842, die US-6 319 232, die EP-0 566 631, die EP-0 566 671, die EP-0 649 382, die EP-0 995 455 und die EP-0 815 888. Bei allen diesen Lösungen ist ein Schutzteil für die Injektionsnadel seitlich wegklappbar.

Bei diesen Lösungen wird der Einrastvorgang, der die Nadel bzw. die Nadelspitze in die Schutzposition bringt, durch einseitigen seitlichen Druck auf das jeweilige Schutzteil, und in der Folge durch das Einklappen des Schutzteiles gegen die Nadel, bewerkstelligt.

Einige Lösungen, wie beispielsweise die US-4 820 277 oder die WO-91/09639, zeigen auch Ausführungsvarianten, bei denen das Schutzteil zweiteilig ist und beide Teile seitlich wegklappbar sind.

Es gibt allerdings auch Lösungen, die keine seitlich wegklappbaren Teile verwenden. So zeigt beispielsweise die US-5 250 031 eine einrastbare Nadelabdeckung mit einem kappenartigen Schutzteil, das über zwei elastische Federarme mit einem Basisteil am Nadelgrund verbunden ist. In der Nadelabdeckung befindet sich ein Einrastmittel in der Form einer Bohrung mit speziell geformtem Einrastweg zur Aufnahme der Nadelspitze in der Schutzposition. Bei dieser Vorrichtung wird der Einrastvorgang, der die Nadelspitze in die Schutzposition bringt, vorzugsweise durch Zusammendrücken der zwei elastischen Federarme ausgelöst. Die Elastizität der Federarme dient hier lediglich dazu, die Nadelspitze im Nichtgebrauchsfall innerhalb der Nadelabdeckung zu positionieren, jeglicher Druck der Injektionsnadel gegen die Haut des Patienten lässt die Nadelspitze jedoch aus der Nadelabdeckung hervorragen. Nachteilig bei dieser Lösung dürfte auch sein, dass es nicht ohne weiteres erkennbar ist, ob sich die Nadel in einem gesicherten Zustand (Schutzposition) befindet.

Aufgabe der Erfindung ist es, eine weitere einfach zu bedienende und zuverlässig wirkende Sicherheitsvorrichtung mit einem seitlich wegklappbaren Schutzteil für eine Injektionsnadel anzugeben.

Diese Aufgabe wird durch die Merkmale des Patentanspruchs 1 gelöst.

Die Lösung besteht darin, dass bei einer gattungsgemässen Sicherheitsvorrichtung für eine Injektionsnadel das Schutzteil zumindest in einem Teilbereich seiner Längserstreckung längselastisch ausgebildet ist. In Zusammenwirkung mit einer mechanisch wirkenden Einrastvorrichtung im Schutzteil, beispielsweise eines überstehenden Sperrteiles, lässt sich auf einfache Weise eine Funktionsweise erzielen, bei der nur bei manuell erzeugter und im wesentlichen maximaler längselastischer Verformung des Schutzteiles eine Freigabe der Injektionsnadel überhaupt möglich ist.

Zur Bewirkung der längselastischer Verformung des Schutzteiles kann dabei beispielsweise eine vorstehende Griffnase am Schutzteil verwendet werden, oder es können auch den längselastischen Teilbereich des Schutzteiles überbrückende Spannbügel verwendet werden. Bei ersterem wird die längselastische Verformung durch Druck auf die vorstehende Griffnase in Richtung zur Nadelspitze, bei letzterem durch Zusammendrücken der zwei Spannbügel, erzeugt. In beiden Fällen kann dies mit einer Hand erfolgen.

Für die Ausgestaltung des längselastischen Teilbereich des Schutzteiles wird vorzugsweise eine ziehharmonikaähnliche oder faltenbalgähnliche Form verwendet. Sie hat den Vorteil, besonders gut in der vorgesehenen einstückigen Bauweise von Basisteil, elastischem Gelenkteil und Schutzteil realisierbar zu sein.

Vorteilhafterweise weist das elastische Gelenkteil auch eine Vorspannung auf, die das Schutzteil bei nicht in der Einrastvorrichtung eingerasteter Injektionsnadel selbsttätig seitlich von der Längsachse der Injektionsnadel wegklappen lässt. Dies ermöglicht, dass das medizinische Personal bei der Ausführung von Injektionen nicht unnötig behindert wird. Zudem ist auch jederzeit ohne weiteres klar erkennbar, ob sich die Nadel in einem gesicherten Zustand (Schutzposition) befindet.

Schliesslich kann das Basisteil auch noch zur drehbaren oder festen Anbringung am Halteteil der Injektionsnadel ausgestaltet sein. Insbesondere die drehbare Anbringung kann dazu dienen, unnötige Behinderungen bei der Handhabung zu vermeiden.

Im folgenden werden zwei Ausführungsbeispiele der Erfindung anhand von Zeichnungen näher erläutert.

In den Zeichnungen zeigen:
- Fig. 1: eine seitliche Teilschnittdarstellung einer Injektionsnadel mit einer Sicherheitsvorrichtung für die Injektionsnadel, wobei sich die Sicherheitsvorrichtung in einer Schutzposition befindet,
- Fig. 2: eine Teilschnittdarstellung der Injektionsnadel mit der Sicherheitsvorrichtung gemäss Fig. 1, wobei sich die Sicherheitsvorrichtung in einer Gebrauchsposition befindet,
- Fig. 3: eine räumliche Ansicht der Sicherheitsvorrichtung gemäss Fig. 1,
- Fig. 4: eine weitere Teilschnittdarstellung der Injektionsnadel mit der Sicherheitsvorrichtung gemäss Fig. 1, und
- Fig. 5: eine räumliche Ansicht einer weiteren Ausführungsform der Sicherheitsvorrichtung für eine Injektionsnadel.

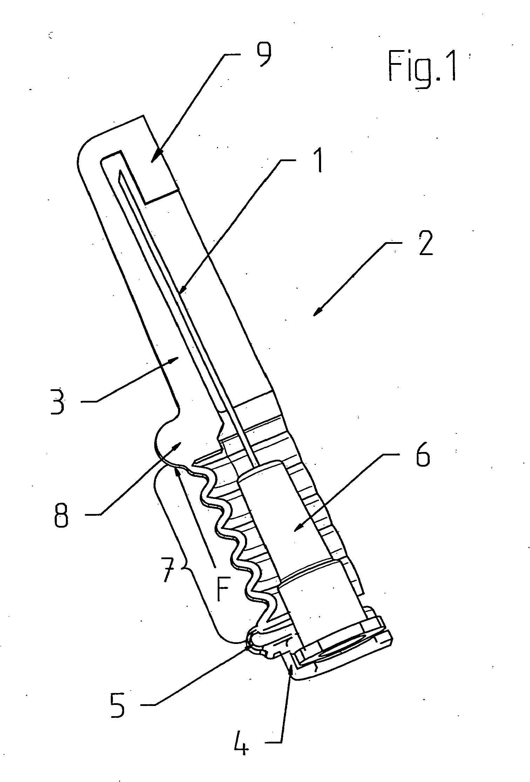

Die Figur 1 zeigt eine seitliche Teilschnittdarstellung einer Injektionsnadel 1 mit einer Sicherheitsvorrichtung 2 für die Injektionsnadel 1, wobei sich die Sicherheitsvorrichtung 2 in einer Schutzposition befindet. In dieser Schutzposition ist die (potentiell gefährliche) Spitze der Injektionsnadel 1 durch ein Schutzteil 3 vor zufälligen Berührungen geschützt.

Die Sicherheitsvorrichtung 2 besteht im wesentlichen aus drei Teilen, nämlich aus einem Basisteil 4, einem elastischen Gelenkteil 5 und dem länglichen Schutzteil 3. Die Sicherheitsvorrichtung 2 ist mittels des Basisteils 4 an einem Halteteil 6 der Injektionsnadel 1 anbringbar.

Die Sicherheitsvorrichtung 2, d.h. das Basisteil 4, das elastische Gelenkteil 5 und das Schutzteil 3 sind dabei vorzugsweise einstückig und aus Kunststoff hergestellt.

Das Schutzteil 3 hat zudem eine mechanisch wirkende Einrastvorrichtung für die Sicherung der Injektionsnadel 1 in der gezeigten Schutzposition, und das Schutzteil 3 ist mittels des elastischen Gelenkteiles 5 seitlich von der Längsachse der Injektionsnadel 1 wegklappbar.

Weiterhin ist das Schutzteil 3 zumindest in einem Teilbereich 7 seiner Längserstreckung längselastisch ausgebildet. Diese längselastische Ausbildung besteht vorzugsweise in einer ziehharmonikaähnlichen oder faltenbalgähnlichen Ausformung. Durch die Ausnützung der natürlichen Elastizität des verwendeten Kunststoffes, beispielsweise von Polyethylen, lässt sich so die gewünschte, längselastische Beweglichkeit leicht erzielen.

Das Schutzteil 3 hat in seinem Teilbereich 7, der für die Längserstreckung längselastisch ausgebildet ist, eine vorstehende Griffnase 8, die zur erleichterten manuellen Erzeugung der längselastischen Verformung mittels einer Schiebebewegung dient. Die Griffnase 8 kann bequem mit dem Daumen einer Hand bedient werden (Ausübung einer Kraft F).

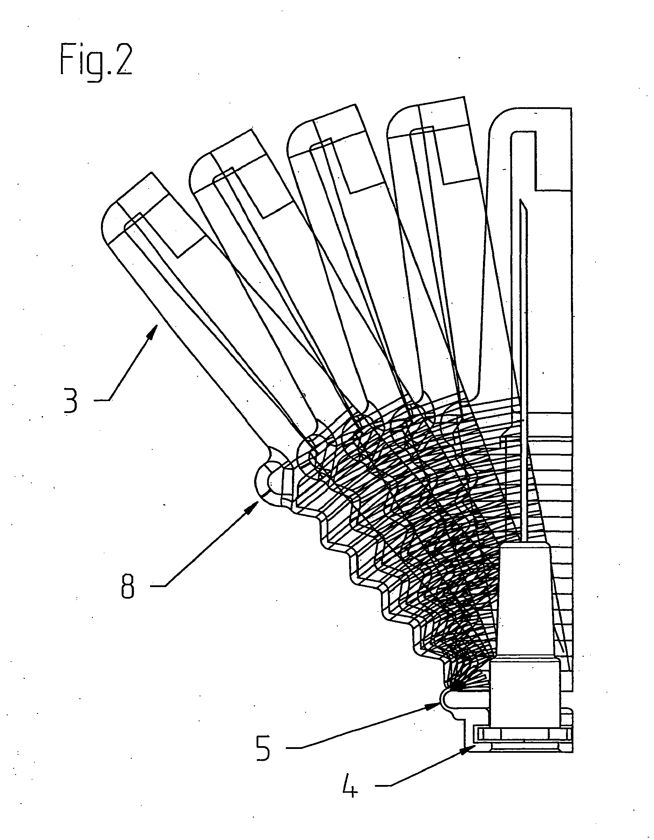

Das elastische Gelenkteil 5 weist vorzugsweise eine Vorspannung auf, die das Schutzteil 3 bei nicht in der Einrastvorrichtung eingerasteter Injektionsnadel 1 selbsttätig seitlich von der Längsachse der Injektionsnadel wegklappen lässt (vgl. dazu Fig. 2).

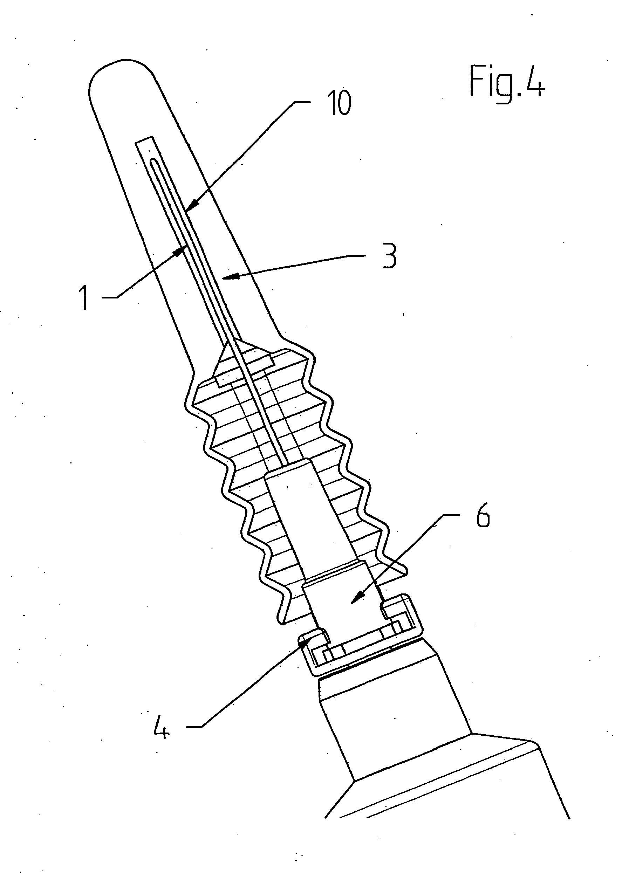

Die bereits erwähnte mechanisch wirkende Einrastvorrichtung ist beispielsweise ein überstehendes Sperrteil 9, dergestalt dass nur bei manuell erzeugter und im wesentlichen maximaler längselastischer Verformung des Schutzteiles 3 eine Freigabe der Injektionsnadel 1 erfolgen kann. Da sich die Injektionsnadel 1 in der dargestellten Schutzposition in einem Aufnahmeschlitz 10 befindet (vgl. dazu auch Fig. 4), bewirkt eine ausgeübte Kraft F, dass sich das Schutzteil 3 elastisch verlängert und die Spitze der Injektionsnadel 1 unter dem überstehenden Sperrteil 9 durchbewegt werden kann. Selbstverständlich kann das Sperrteil 9 auch anders ausgeformt sein; beispielsweise genügt auch ein durchgehender Bolzen oder Zapfen auf der Höhe des Sperrteiles 9.

Schliesslich kann das Basisteil 4 entweder drehbar oder auch fest am Halteteil 6 der Injektionsnadel 1 angebracht sein.

Die Figur 2 zeigt eine Teilschnittdarstellung der Injektionsnadel 1 mit der Sicherheitsvorrichtung 2 gemäss Fig. 1, wobei sich die Sicherheitsvorrichtung 2 hier in einer Gebrauchsposition befindet. Das elastischen Gelenkteil 5 lässt dabei infolge einer Vorspannung das Schutzteil 3 selbsttätig seitlich von der Längsachse der Injektionsnadel 1 wegklappen. In der Gebrauchsposition ist die (potentiell gefährliche) Spitze der Injektionsnadel 1 nicht mehr durch das Schutzteil 3 vor zufälligen Berührungen geschützt.

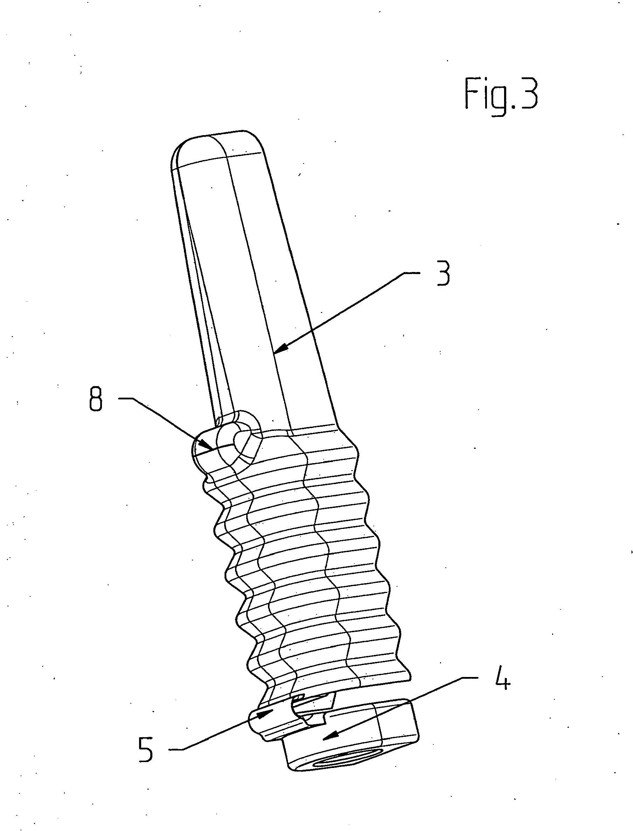

Die Figur 3 zeigt eine räumliche Ansicht der Sicherheitsvorrichtung 2 gemäss Fig. 1.

Die Figur 4 zeigt eine weitere Teilschnittdarstellung der Injektionsnadel 1 mit der Sicherheitsvorrichtung 2 gemäss Fig. 1, hier in einer Schnittebene rechtwinklig zu derjenigen von Fig. 1. Die Sicherheitsvorrichtung 2 ist hier auch in der Schutzposition und die Injektionsnadel 1 befindet sich im Aufnahmeschlitz 10.

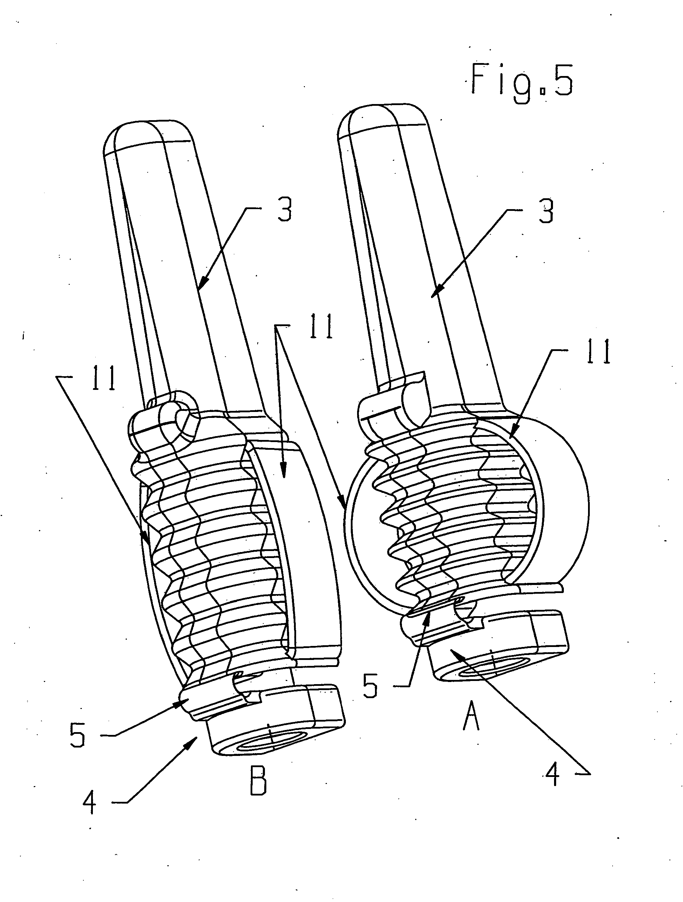

Die Figur 5 zeigt eine räumliche Ansicht einer weiteren Ausführungsform der Sicherheitsvorrichtung 2 für eine Injektionsnadel 1. Bei dieser Ausführungsform fehlt die Griffnase 8, dafür hat das Schutzteil 3 in seinem Teilbereich 7, der für die Längserstreckung längselastisch ausgebildet ist, zwei gegenüberliegende, bogenförmige und den längselastischen Teilbereich überbrückende Spannbügel 11. Die längselastische Verformung des Schutzteils 3 kann hier durch manuelles gegenseitiges Zusammendrücken der zwei Spannbügel 11 bewirkt werden, was natürlich zwischen Daumen und Zeigefinger einer Hand erfolgen kann.

Die Fig. 5 zeigt ferner einen Zustand A, bei dem die zwei Spannbügel 11 nicht zusammengedrückt sind und das Schutzteil 3 somit seine normale Längsausdehnung hat, sowie einen Zustand B, bei dem die zwei Spannbügel zusammengedrückt sind und das Schutzteil 3 somit eine grössere Längsausdehnung als im Zustand A aufweist.

Bezugsziffernliste:
- 1: Injektionsnadel
- 2: Sicherheitsvorrichtung (für eine Injektionsnadel)
- 3: Schutzteil
- 4: Basisteil
- 5: Gelenkteil
- 6: Halteteil (der Injektionsnadel)
- 7: Teilbereich (längselastischer)
- 8: Griffnase
- 9: Sperrteil
- 10: Aufnahmeschlitz
- 11: Spannbügel

- F: Kraft

## Patentansprüche

1. Sicherheitsvorrichtung (2) für eine Injektionsnadel (1) mit einem an einem Halteteil (6) der Injektionsnadel (1) anbringbaren Basisteil (4) und einem über ein elastisches Gelenkteil (5) mit dem Basisteil (4) verbundenen länglichen Schutzteil (3), wobei das Basisteil (4), das elastische Gelenkteil (5) und das Schutzteil (3) einstückig und aus Kunststoff hergestellt sind, das Schutzteil (3) eine mechanisch wirkende Einrastvorrichtung für die Sicherung der Injektionsnadel (1) in einer Schutzposition aufweist und das Schutzteil (3) mittels des elastischen Gelenkteiles (5) seitlich von der Längsachse der Injektionsnadel (1) wegklappbar ist, **dadurch gekennzeichnet, dass** das Schutzteil (3) zumindest in einem Teilbereich (7) seiner Längserstreckung längselastisch ausgebildet ist.

2. Sicherheitsvorrichtung (2) nach Patentanspruch 1, **dadurch gekennzeichnet, dass** der Teilbereich (7) des Schutzteiles (3), der in seiner Längserstreckung längselastisch ausgebildet ist, eine ziehharmonikaähnliche oder faltenbalgähnliche Ausbildung aufweist.

3. Sicherheitsvorrichtung (2) nach Patentanspruch 1 oder 2, **dadurch gekennzeichnet, dass** das Schutzteil (3) in seinem Teilbereich (7), der für die Längserstreckung längselastisch ausgebildet ist, eine vorstehende Griffnase (8) aufweist, die zur erleichterten manuellen Erzeugung der längselastischen Verformung mittels einer Schiebebewegung dient.

4. Sicherheitsvorrichtung (2) nach Patentanspruch 1 oder 2, **dadurch gekennzeichnet, dass** das Schutzteil (3) in seinem Teilbereich (7), der für die Längserstreckung längselastisch ausgebildet ist, zwei gegenüberliegende, bogenförmige und den längselastischen Teilbereich (7) überbrückende Spannbügel (11) aufweist, die zur erleichterten manuellen Erzeugung der längselastischen Verformung mittels einer Zusammendrückbewegung dienen.

5. Sicherheitsvorrichtung (2) nach einem der Patentansprüche 1 bis 4, **dadurch gekennzeichnet, dass** das elastische Gelenkteil (5) eine Vorspannung aufweist, die das Schutzteil (3) bei nicht in der Einrastvorrichtung eingerasteter Injektionsnadel (1) selbsttätig seitlich von der Längsachse der Injektionsnadel (1) wegklappen lässt.

6. Sicherheitsvorrichtung (2) nach einem der Patentansprüche 1 bis 5, **dadurch gekennzeichnet, dass** die mechanisch wirkende Einrastvorrichtung ein überstehendes Sperrteil (9) ist, dergestalt dass nur bei manuell erzeugter und im wesentlichen maximaler längselastischer Verformung des Schutzteiles (3) eine Freigabe der Injektionsnadel (1) erfolgt.

7. Sicherheitsvorrichtung (2) nach einem der Patentansprüche 1 bis 6, **dadurch gekennzeichnet, dass** das Basisteil (4) drehbar oder fest am Halteteil (6) der Injektionsnadel (1) anbringbar ist.
